# EUROPEAN PATENT APPLICATION

(11) **EP 4 574 997 A1**
(43) Date of publication of application: **25.06.2025**
(21) Application number: 23218158.6
(22) Date of filing: 19.12.2023
(51) Int. Cl.: C12Q 1/6886

(54) **PREDICTION OF AN OUTCOME OF A PROSTATE CANCER SUBJECT**

(71) Applicant: Koninklijke Philips N.V., 5656 AG Eindhoven (NL); The University Court of the University of Glasgow, Glasgow G12 8QQ (GB)
(72) Inventor: HOFFMANN, Ralf Dieter, Eindhoven (NL); BAILLIE, George, Eindhoven (NL)
(74) Representative: Philips Intellectual Property & Standards

(57) **Abstract**

The invention relates to methods for predicting an outcome in a prostate cancer patient. The method is based on determining or receiving information about the transcription level of one or more transcript of the PDE4D genomic region or the DNA methylation of the PDE4D genomic region. The method may be used to predict an outcome such as likelihood of or time to biochemical recurrence. The invention further relates to products for determining transcription levels of non-coding RNAs in the PDE4D genomic region and uses thereof.

## Description

### FIELD OF THE INVENTION

The invention relates to methods for predicting an outcome for a patient with prostate cancer. In particular the invention relates to determining the transcription level of one or more non-coding RNAs in the genomic region of the PDE4D gene on the human genome, or DNA methylation levels in the PDE4D genomic region. The invention further relates to products for determining the transcription levels of one or more transcripts from the PDE4D genomic region.

### BACKGROUND OF THE INVENTION

Given the substantial heterogeneity in prostate cancer (PCa) treatment responses vary, therefore a prognostic biomarker indicating disease progression would influence personalized treatment decisions and identify novel therapeutic targets. Dysregulated cyclic AMP (cAMP) signaling is associated with cancer, particularly in the progression from localized androgen-sensitive (AS) PCa to aggressive castration-resistant PCa (CRPC). cAMP signaling depends on the compartmentalization of cAMP effector proteins and phosphodiesterases (PDEs). The PDE4D sub-family is germane in PCa, with the isoform PDE4D7 being of unique importance. Interestingly, PDE4D7 expression correlates inversely with disease progression, with downregulation conferring an increased risk of disease recurrence. This highlights the potential of PDE4D7 as a prognostic biomarker to distinguish between insignificant and aggressive tumors, as well as representing potential novel therapeutic avenues.

Although a useful biomarker, there is an ever existing need to provide alternative biomarkers for predicting, diagnosing and stratifying patients suffering from cancer. For example, depending on the sequencing conditions used it may not be possible to distinguish between expression levels of individual PDE4D transcripts, and thus not possible to provide PDE4D isoform 7 specific transcription levels, only overall PDE4D transcription levels. Other biomarkers may thus provide an alternative for such cases, or even provide an improved prediction even if PDE4D7 specific expression levels are available.

The present invention aims to provide methods and uses as defined in the appended claims which provide such alternative or improved prediction.

### SUMMARY OF THE INVENTION

In a first aspect the invention relates to a computer implemented method for predicting an outcome for a human prostate cancer patient, the method comprising a) receiving information on the transcription level of at least one transcript from the genomic region of the human chromosome 5 bases 59,000,000 to 60,600,000 with the proviso that the transcript is not PDE4D or a specific PDE4D isoform, and/or receiving information on the DNA methylation level in a genomic region of the human chromosome 5 bases 58,900,000 to 61,000,000, determined in a sample obtained from the patient, b) comparing the transcription level and/or DNA methylation level to a reference, and c) predicting a poor outcome if transcription levels are below the reference value and/or DNA methylation levels are above the reference value, or predicting a favorable outcome if transcription levels are above the reference value and/or DNA methylation levels are below the reference value.

In a second aspect the invention relates to a method for determining an outcome for a human prostate cancer patient, the method comprising a) determining the transcription level of at least one transcript from the genomic region of the human chromosome 5 bases 59,000,000 to 60,600,000 with the proviso that the transcript is not PDE4D or a specific PDE4D isoform, and/or determining the DNA methylation level in a genomic region of the human chromosome 5 bases 58,900,000 to 61,000,000, determined in a sample obtained from the patient, b) comparing the transcription level and/or DNA methylation level to a reference, and c) predicting a poor outcome if transcription levels are below the reference value and/or DNA methylation levels are above the reference value, or predicting a favorable outcome if transcription levels are above the reference value and/or DNA methylation levels are below the reference value.

In a third aspect the invention relates to a kit of parts comprising primers and optionally probes for the selective amplification and detection of least one transcript is selected from NDUFB4P2, MIR582, AC034234.1, RNU6-806P, AC109486.1, SETP21, and PART1, preferably selected from AC034234.1, RNU6-806P, and SETP21.

In a fourth aspect the invention relates to the use of the kit according to the third aspect of the invention for predicting an outcome for a human prostate cancer patient, the use comprising: determining the expression level of the at least one transcript using the kit; and predicting the outcome based on the at least one expression level.

### BRIEF DESCRIPTION OF THE DRAWINGS

Embodiments of the invention are further described hereinafter with reference to the accompanying drawings, in which:

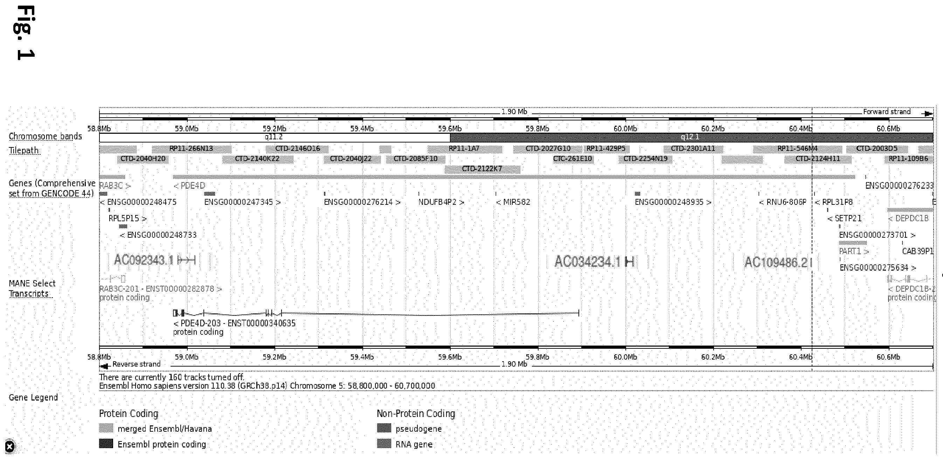

Fig. 1 depicts a schematic overview of the PDE4D genomic region on the human chromosome. Depicted is a genome browser view of human Chromosome 5 bases 58.800.000 to 60.600.000, indicated are the PDE4D gene and non-coding transcripts NDUFB4P2, MIR582, AC034234.1, RNU6-806P, AC109486.1, SETP21, and PART1.

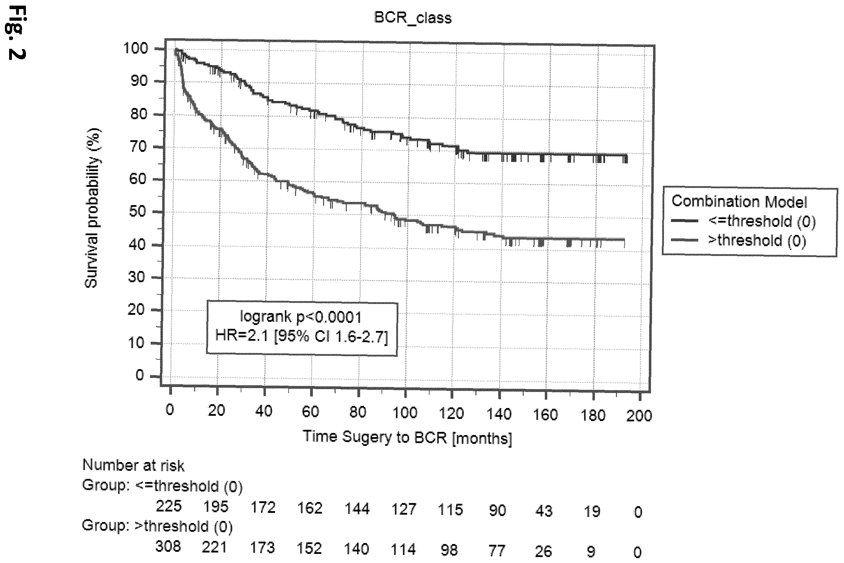

Fig 2. depicts Kaplan-Meier survival curve analysis of the combined model. The clinical endpoint tested was time to biochemical recurrence in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set and were fixed for validation in the test cohort (shown). In the validation (test) cohort, the combined model predicts time to biochemical recurrence; patients classified into the high-risk group (>threshold 0) had a 2.1-fold increased hazard risk to reach biochemical recurrence.

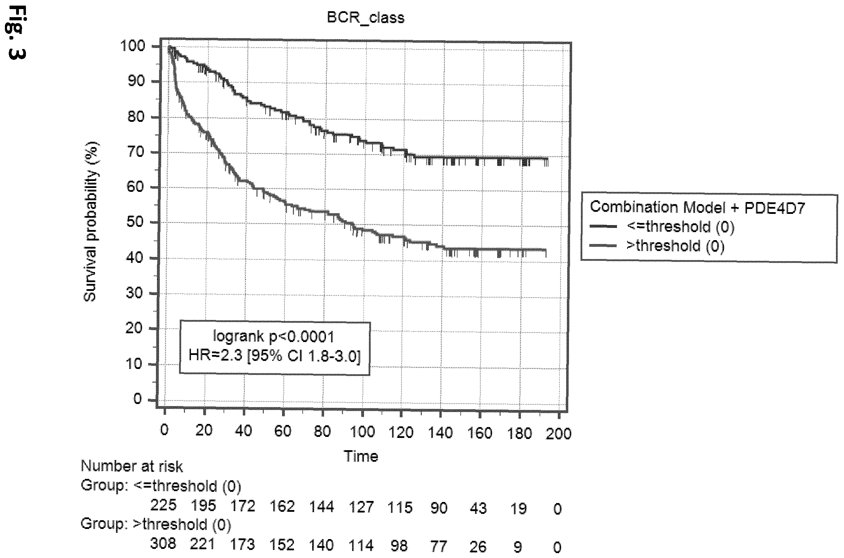

Fig 3. depicts Kaplan-Meier survival curve analysis of the combined model + PDE4D7. The clinical endpoint tested was time to biochemical recurrence in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set and were fixed for validation in the test cohort (shown). In the validation (test) cohort, the combined model + PDE4D7 predicts time to biochemical recurrence; patients classified into the high-risk group (>threshold 0) had a 2.3-fold increased hazard risk to reach biochemical recurrence.

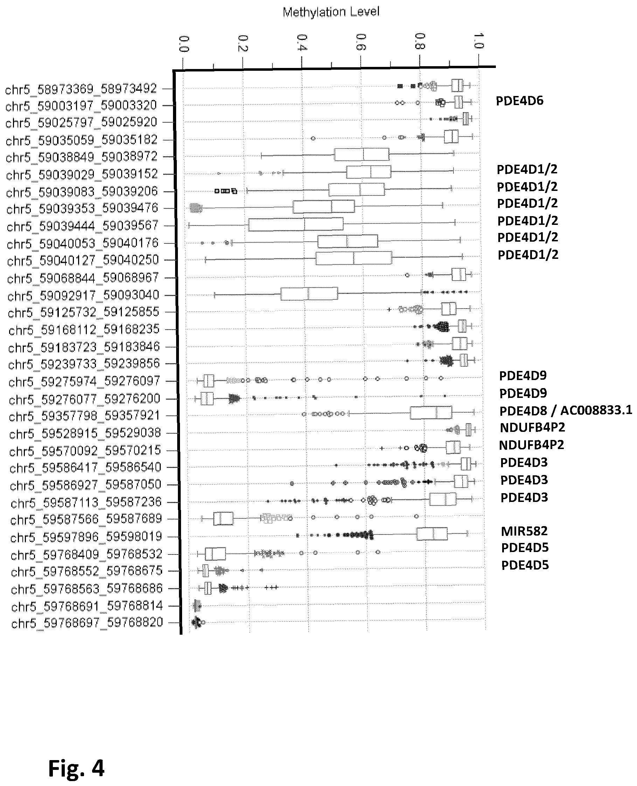

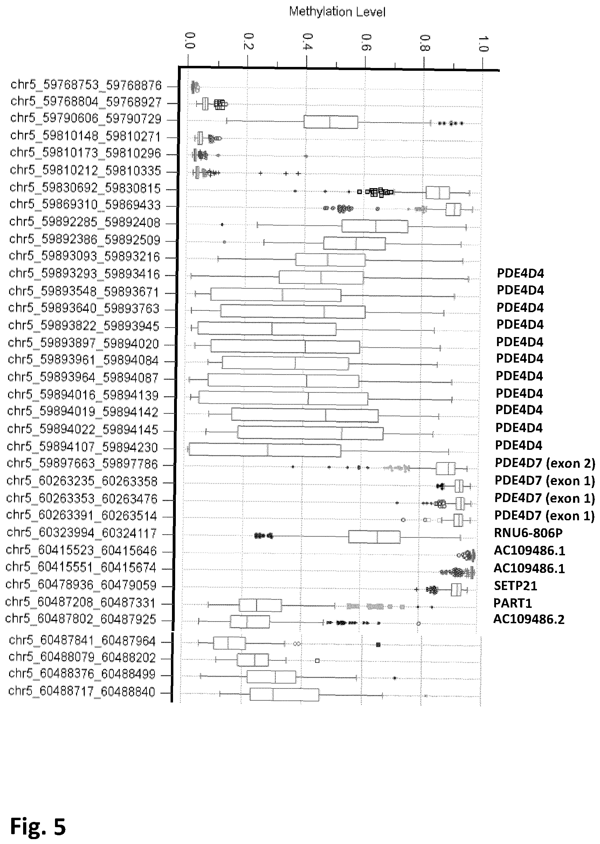

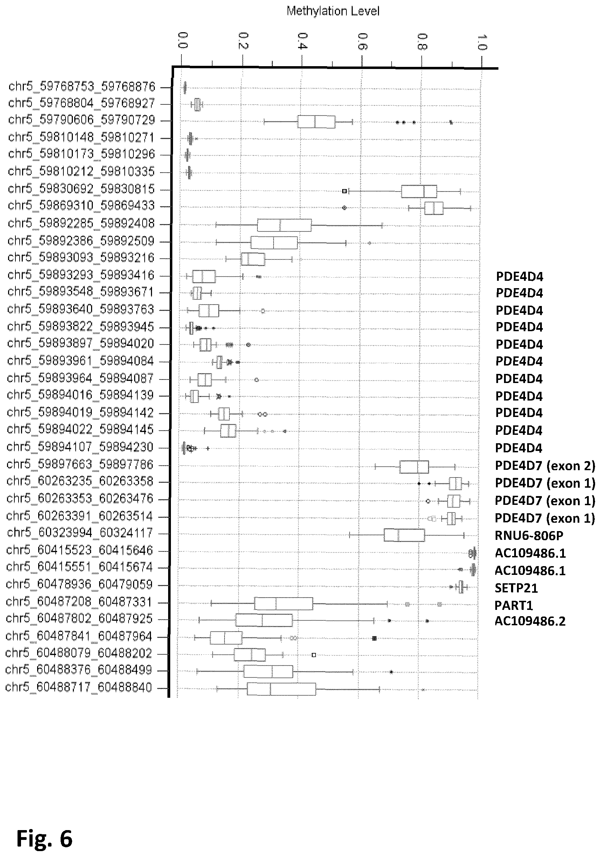

Fig. 4 to 7 depict data of primary tumor samples (Fig. 4 and 5) and for the normal samples (Fig. 6 and 7). Shown on the y-axis are the genome positions (start to end) of the probe sets. On the x-axis is shown the so-called beta-values which is a measure between 0-1 where 0 means no methylation and 1 means complete methylation of the GC site that is measured by the respective probe. The box plots represent the beta values for the ca 500 primary tumor samples (Figs. 4 and 5) and the ca 50 normal prostate samples (Figs. 6 and 7). Also indicated are the next probe set(s) for the features of interest in the PDE4D region

### DEFINITIONS

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. For example, a method for administrating a pharmaceutical agent includes the administrating of a plurality of molecules (e.g., 10's, 100's, 1000's, 10's of thousands, 100's of thousands, millions, or more molecules).

As used herein, "about" and "approximately", when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed invention. Unless otherwise clear from context, all numerical values provided herein include numerical values modified by the term "about."

As used herein, "and/or" refers to a situation wherein one or more of the stated cases may occur, alone or in combination with at least one of the stated cases, up to with all of the stated cases.

As used herein, "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

As used herein, "conventional techniques" or "methods known to the skilled person" refer to a situation wherein the methods of carrying out the conventional techniques used in methods of the invention will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, "exemplary" or "for example" means "serving as an example, instance, or illustration," and should not be construed as excluding other configurations, including those disclosed herein.

Throughout this disclosure, various aspects of the invention can be presented in a range format. It should be understood that the description in range format is merely for convenience and should not be construed as a limitation on the scope of the invention. The description of a range should be considered to have specifically disclosed all the possible subranges as well as individual numerical values within that range including both integers and non-integers. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, 6 etc. This applies regardless of the breadth of the range.

As used herein, "cancer" refers to the physiological condition in mammals that is typically characterized by unregulated cell growth. The terms "cancer," "neoplasm," and "tumor," are often used interchangeably to describe cells that have undergone a malignant transformation that makes them pathological to the host organism. Primary cancer cells can be distinguished from non-cancerous cells by techniques known to the skilled person. A cancer cell, as used herein, includes not only primary cancer cells, but also cancer cells derived from such primary cancer cell, including metastasized (secondary) cancer cells, and cell lines derived from cancer cells. Examples include solid tumors and non-solid tumors or blood tumors. Treatment of a cancer in a subject includes the treatment of a tumor in the subject.

The term "prostate cancer" refers to a cancer of the prostate tissue, which occurs when cells in the prostate mutate and begin to grow out of control.

The term "prostate cancer specific death or disease specific death" refers to death of a patient from a prostate cancer.

The term "clinical recurrence" refers to the presence of clinical signs indicating the presence of tumor cells as measured, for example using in vivo imaging.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

The term "metastases" refers to the presence of metastatic disease in organs other than a bladder tissue.
intramuscular, or intratumoral delivery or injection.

As used herein, the term "pharmaceutical composition" refers to a composition formulated in pharmaceutically acceptable or physiologically-acceptable compositions for administration to a cell or subject. The compositions of the invention may be administered in combination with other agents as well, provided that the additional agents do not adversely affect the ability of the composition to deliver the intended therapy. The pharmaceutical composition often comprise, in addition to a pharmaceutical active agent, one or more pharmaceutical acceptable carriers (or excipients).

As used herein, a "subject" is to indicate the organism to be treated e.g., to which administration is contemplated. The subject may be any subject in accordance with the present invention, including, but not limited to humans, males, females, infants, children, adolescents, adults, young adults, middle-aged adults or senior adults and/or other primates or mammals. Preferably the subject is a human patient. In some embodiments, the subject may have been diagnosed with cancer, an immune related disorder, a bleeding disorder, a disorder related to over expression of a protein, a disorder related to under expression of a protein. In some embodiments the subject may be at risk of developing a disease or disorder which may be prevented or ameliorated by vaccination.

As used herein, a "T-cell" can be selected from, for example, the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. They can be extracted from blood or derived from stem cells. T-cells can be obtained from a number of non-limiting sources, including peripheral blood mononuclear cells, bone marrow, lymph node tissue, cord blood, and tumors. In certain embodiments of the present invention, any T cell line available and known to those skilled in the art, may be used. In another embodiment, said cell can be derived from a healthy donor, from a patient diagnosed with cancer or from a patient diagnosed with an infection. In another embodiment, said cell is part of a mixed population of cells which present different phenotypic characteristics.

As used herein, "treatment", "treating", "palliating", "alleviating" and "ameliorating" in the context of a subject to be treated, all refer to an approach for obtaining beneficial or desired results including, but not limited to, therapeutic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. As used herein, "prevention" and "preventing" refers to an approach for reducing in part or in full the change of developing adverse effects, for example normally associated with the use of a particular drug or agent. Within the context of the current invention, for example, the terms may refer to preventing, treating or reducing the effects of cancer, an immune related disorder, a bleeding disorder, a disorder related to over expression of a protein, a disorder related to under expression of a protein. In some embodiments the preventing may also refer to the effects of preventing a disease by vaccination.

As used herein the term "nucleic acid" or "polynucleotide" refers to any polymers or oligomers of (contiguous) nucleotides. The nucleic acid may be DNA or RNA, or a mixture thereof, and may exist permanently or transitionally in single-stranded or double-stranded form, including homoduplex, heteroduplex, and hybrid states. The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. The term "isolated" thus means isolated from naturally occurring sources or artificially or synthetically produced.

As used herein, "Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, the terms "protein" and "polypeptide" refer to molecules consisting of a chain of amino acids, without reference to a specific mode of action, size, three dimensional structure or origin. A "fragment" or "portion" or "part" of a polypeptide may thus still be referred to as a "polypeptide". An "isolated protein" or isolated polypeptide" is used to refer to a protein or polypeptide which is no longer in its natural environment, for example in vitro or in a recombinant host cell.

As used herein the term "construct" or "nucleic acid construct" or "vector" refers to a man-made nucleic acid molecule resulting from the use of recombinant DNA technology and which is used to deliver exogenous DNA into a host cell, often with the purpose of expression in the host cell of a DNA region comprised on the construct. The vector backbone of a construct may for example be a plasmid into which a (chimeric) gene is integrated or, if a suitable transcription regulatory sequence is already present, only a desired nucleic acid sequence (e.g. a coding sequence) is integrated downstream of the transcription regulatory sequence. Vectors may comprise further genetic elements to facilitate their use in molecular cloning, such as e.g. selectable markers, multiple cloning sites and the like.

### DETAILED DESCRIPTION OF THE INVENTION

The invention is defined herein, and in particular in the accompanying claims. Subject-matter which is not encompassed by the scope of the claims does not form part of the present claimed invention.

It is contemplated that any method, use or composition described herein can be implemented with respect to any other method, use or composition described herein. Embodiments or preferences discussed in the context of methods, use and/or compositions of the invention may likewise be employed with respect to any other method, use or composition described herein. Thus, an embodiment or preference pertaining to one method, use or composition may be applied to other methods, uses and compositions of the invention as well.

Any references in the description to methods of treatment refer to the compounds, pharmaceutical compositions and medicaments of the present invention for use in a method for treatment of the human (or animal) body by therapy.

PDE4D7 expression is diminished during progression of PCa and the phosphodiesterase has been proposed as a prognostic biomarker. RNA sequencing of PCa tissue identified sequences in the PDE4D coding region on Chr5q12 exhibiting similarities in mRNA expression profile to PDE4D7. Several transcripts in the genomic region matched expression to PDE4D7, including miRNAs and IncRNAs. These novel PDE4D7 associated genes represent putative PCa biomarkers and could have mechanistic roles in PCa progression.

As embodied and broadly described herein, the present invention is directed to determining the expression of a transcript in the genomic region of the PDE4D7 encoding gene, and using said transcription to predict an outcome for a subject having prostate cancer. As it is well appreciated that silencing (or upregulation) of the transcription of an entire genomic locus is likely caused be DNA methylation of the locus, the invention further extends to determining the DNA methylation state of the genomic region of the PDE4D7 transcript.

Therefore, in a first aspect the invention relates to a computer implemented method for predicting an outcome for a human prostate cancer patient, the method comprising a) receiving information on the transcription level of at least one transcript from the genomic region of the human chromosome 5 bases 59,000,000 to 60,600,000 with the proviso that the transcript is not PDE4D or a specific PDE4D isoform, and/or receiving information on the DNA methylation level in a genomic region of the human chromosome 5 bases 58,900,000 to 61,000,000, determined in a sample obtained from the patient, b) comparing the transcription level and/or DNA methylation level to a reference, and c) predicting a poor outcome if transcription levels are below the reference value and/or DNA methylation levels are above the reference value, or predicting a favorable outcome if transcription levels are above the reference value and/or DNA methylation levels are below the reference value.

Alternatively, the invention relates to a method for determining an outcome for a human prostate cancer patient, the method comprising a) determining the transcription level of at least one transcript from the genomic region of the human chromosome 5 bases 59,000,000 to 60,600,000 with the proviso that the transcript is not PDE4D or a specific PDE4D isoform, and/or determining the DNA methylation level in a genomic region of the human chromosome 5 bases 58,900,000 to 61,000,000, determined in a sample obtained from the patient, b) comparing the transcription level and/or DNA methylation level to a reference, and c) predicting a poor outcome if transcription levels are below the reference value and/or DNA methylation levels are above the reference value, or predicting a favorable outcome if transcription levels are above the reference value and/or DNA methylation levels are below the reference value.

The method is thus based on obtaining information on at least transcription levels in the genomic region and/or DNA methylation level in the genomic region. It is understood that information on the transcription levels(s) and/or DNA methylation may be determined independently and provided to perform the analysis, or obtaining the information may form part of method.

The present invention is based in the insight of the inventors that not only PDE4D or specific isoforms such as PDE4D7 are informative for prostate cancer outcome, but that the whole genomic region surrounding the PDE4D gene shows different transcription of non-coding RNAs and differences in methylation. The genomic region of the human PDE4D gene on chromosome 5 is schematically depicted in Fig. 1. Examples 1 and 2 show data that individual non-coding transcripts located in this genomic region are informative for predicting an outcome in prostate cancer. Furthermore Example 3 provides evidence of distinct DNA methylation between primary prostate tumors and healthy tissue, and makes it plausible that an outcome for prostate cancer can be predicted based on a combination of specific methylation markers in the PDE4D4 genomic area or overall DNA methylation levels.

The method broadly described herein is used to predict an outcome for a prostate cancer patient. The method is based on information retrieved from a sample of the patient. The outcome refers to a predicted progression of the disease based on the status of the patient when the sample was obtained, and may for example predict disease progression, cancer or recurrence free survival time, time to recurrence, e.g. specifically to biochemical recurrence, 5-year survival rate, 10-year survival rate or 15-year survival rate, time to death or time to prostate cancer specific death.

The sample obtained from the patient is preferably a sample comprising at least one tumor cell. Therefore the sample may for example be a tumor biopsy, a biopsy of a metastasis, a blood sample comprising circulating tumor cells, or a plasma or urine sample comprising tumor cells.

Transcription levels of transcripts, particularly non-coding transcripts can be determined using techniques known to the skilled person, such as but not limited to qPCR, RNA sequencing and microarray technologies. The invention is not limited to a specific technology and can be implemented with any technology that allows quantification of a transcript level in a sample. Preferred technologies are qPCR or RNA sequencing as they generally allow accurate and reproducible quantification.

When used herein the PDE4D genomic region refers to the genomic region which comprises the PDE4D gene and surrounding areas. In the human genome, the PDE4D gene is located at chromosome 5 approximately between bases 58,980,000 and 60,500,000. When referring to a specific location on the human genome the location indicates the coordinates as used in version GRCh38/hg38 of the human genome. Thus in an embodiment the invention relates to a method for predicting an outcome for a human prostate cancer patient, the method comprising a) receiving or detecting information on the transcription level of at least one transcript from the genomic region of the human chromosome 5 bases 59,000,000 to 60,600,000 of version GRCh38/hg38 of the human genome with the proviso that the transcript is not PDE4D or a specific PDE4D isoform, and/or receiving information on the DNA methylation level in a genomic region of the human chromosome 5 bases 58,900,000 to 61,000,000 of version GRCh38/hg38 of the human genome, determined in a sample obtained from the patient, b) comparing the transcription level and/or DNA methylation level to a reference, and c) predicting a poor outcome if transcription levels are below the reference value and/or DNA methylation levels are above the reference value, or predicting a favorable outcome if transcription levels are above the reference value and/or DNA methylation levels are below the reference value.

Within the region of the human chromosome 5 bases 59,000,000 to 60,600,000, besides the PDE4D gene, several non-coding transcripts are located. The inventors herein present evidence that these transcripts are also predictive of an outcome for a patient having a prostate cancer, for example a patient with primary localized disease, locally or regionally advanced disease, or metastatic disease. The inventors present in Example 2 several non-coding transcript in this region that statistically correlated with biochemical recurrence. Thus these transcripts can each individually be used for predicting an outcome for a prostate cancer patient, for example the chance of biochemical recurrence. In addition such predictions may be improved by combining information of multiple transcripts, as for example shown in the combination model. Therefore in a preferred embodiment the method is based on receiving information on the transcription level or determining the transcription level of at least two, preferably three, four, five, six or seven transcripts within the region of the human chromosome 5 bases 59,000,000 to 60,600,000. Preferably the genomic location corresponds to version GRCh38/hg38 of the human genome. Therefore the PDE4D genomic region, when referring to non-coding transcripts that are predictive for a prostate cancer patient outcome is understood to be the region of the human chromosome 5 bases 59,000,000 to 60,600,000.

Because coordinated downregulation is usually indicative of DNA methylation, the inventors proceeded to test if indeed methylation of the PDE4D genomic region is also predictive for an outcome of a prostate cancer patient, e.g. the chance to biochemical recurrence. These results are presented in Example 3 and provide compelling evidence that indeed individual methylation markers, a combination of methylation markers or overall methylation state of the genomic region can be used to predict an outcome. For this the genomic region of the human chromosome 5 bases 58,900,000 to 61,000,000 was analysed and significant difference were found in methylation levels between healthy tissue and primary tumor samples. Therefore the PDE4D genomic region, when referring to DNA methylation sites that are predictive for a prostate cancer patient outcome is understood to be the region of the human chromosome 5 bases 58,900,000 to 61,000,000. Thus in an embodiment DNA methylation level in the genomic region of the human chromosome 5 bases 58,900,000 to 61,000,000 may refer to the methylation level of a single GC site, a combination of GC sites or overall methylation in the PDE4D genomic region (e.g. the region of the human chromosome 5 bases 58,900,000 to 61,000,000).

The present method compares the transcription level or the DNA methylation level of the PDE4D genomic region with a reference to determine if the levels are elevated or decreased or similar to the reference. The reference may be a transcription level or DNA methylation level of a sample form a patient with a known outcome, e.g. a poor outcome or a good outcome. The reference may also be a threshold which is determined in a cohort of samples from patients with both poor and good outcomes, and wherein the threshold is set to distinguish between poor and good outcomes. For example by comparing the transcription level (of one or more transcripts of the PDE4D genomic region) of multiple prostate cancer patients with a poor and a good outcome, statistical methods can be used to determine a threshold above which the likelihood is largest that the outcome is good and below which the likelihood is largest that the outcome is poor. The same can be done for methylation levels, e.g. methylation level of a single GC site, a combination of GC sites or overall methylation in the PDE4D genomic region. Thus the reference may be a single sample, a combination of samples or a threshold.

The methods as broadly described herein are preferably used when the human prostate cancer patient is a patient with primary localized disease, locally or regionally advanced disease, or metastatic disease. Thus in a preferred embodiment the patient has not yet received a treatment. In a preferred embodiment the human prostate cancer patient is a patient with primary localized disease, locally or regionally advanced disease, or metastatic disease.

The method is used for predicting an outcome for a patient with prostate cancer. This outcome may be poor or favorable (used herein interchangeably with good outcome). The outcome generally refers to an endpoint and the expected time to occur or the overall chance to occur. For example the outcome may be biochemical recurrence and the method may predict the chance of biochemical recurrence occurring in the patient or the expected average time to biochemical recurrence in the patient. Other endpoints are for example but not limited to: chance of occurrence of castration resistant prostate cancer, or chance of disease recurrence.

The inventors have identified the non-coding RNAs NDUFB4P2, MIR582, AC034234.1, RNU6-806P, AC109486.1, SETP21, and PART1 in the PDE4D genomic region and found that each of these transcripts individually statistically correlate with disease outcome, and consequently can be used to predict an outcome for a prostate cancer patient. Thus in an embodiment the at least one transcript is selected from NDUFB4P2, MIR582, AC034234.1, RNU6-806P, AC109486.1, SETP21, and PART1, preferably wherein the method comprises determining or receiving the transcription levels of at least two, preferably three, four, five, six, or all seven transcripts selected from NDUFB4P2, MIR582, AC034234.1, RNU6-806P, AC109486.1, SETP21, and PART1.

The term "NDUFB4P2" refers to the NADH:Ubiquinone Oxidoreductase Subunit B4 Pseudogene 2 (Ensembl: ENSG00000251306), for example, to the sequence as defined in transcript no: ENST00000509515.1, specifically, to the nucleotide sequence as set forth in SEQ ID NO:1, which corresponds to the sequence of the above Ensembl Sequence of the NDUFB4P2 transcript.

The term "NDUFB4P2" also comprises nucleotide sequences showing a high degree of homology to NDUFB4P2, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 1.

The term "MIR582" refers to Homo sapiens microRNA 582 (HGNC:32838 or miRBase:MI0003589), for example, to the sequence as defined in GenBank accession no: NR_030308.1, specifically, to the nucleotide sequence as set forth in SEQ ID NO:2, which corresponds to the sequence of the above HGNC Sequence of the MIR582 transcript.

The term "MIR582" also comprises nucleotide sequences showing a high degree of homology to MIR582, e.g. nucleic acid sequences being at least 75%, 80%, 85%, 90%, 91%, 92%, 93%, 94%, 95%, 96%, 97%, 98% or 99% identical to the sequence as set forth in SEQ ID NO:2.

The term "AC034234.1" refers to the non-coding RNA (Ensembl: ENSG00000248935), for example, the sequence as defined in transcript no: ENST00000508696.1 (transcript 1) or ENST00000502993.1 (transcript 2), specifically, to the nucleotide sequence as set forth in SEQ ID NO:3 (transcript 1) or SEQ ID NO:4 (transcript 2), which corresponds to the sequences of the above Ensembl Sequence of the AC034234.1 transcript.

The term "AC034234.1" also comprises nucleotide sequences showing a high degree of homology to AC034234.1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:3 or 4.

The term "RNU6-806P" refers to the RNA, U6 small nuclear 806, pseudogene (Ensembl: ENSG00000202017), for example, to the sequence as defined in transcript no: ENST00000365147.1, specifically, to the nucleotide sequence as set forth in SEQ ID NO:5, which corresponds to the sequence of the above Ensembl Sequence of the RNU6-806P transcript.

The term "RNU6-806P" also comprises nucleotide sequences showing a high degree of homology to RNU6-806P, e.g. nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "AC109486.1" refers to the non-coding transcript (Ensembl: ENSG00000273701), for example, to the sequence as defined in transcript no:
ENST00000611761.1, specifically, to the nucleotide sequence as set forth in SEQ ID NO:6, which corresponds to the sequence of the above Ensembl Sequence of the AC109486.1 transcript.

The term "AC109486.1" also comprises nucleotide sequences showing a high degree of homology to AC109486.1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:6.

The term "SETP21" refers to the prostate androgen-regulated transcript 1 (Ensembl: ENSG00000152931), for example, to the sequence as defined in transcript no: ENST00000506884.2, specifically, to the nucleotide sequence as set forth in SEQ ID NO:7, which corresponds to the sequence of the above Ensembl Sequence of the SETP21 transcript.

The term "SETP21" also comprises nucleotide sequences showing a high degree of homology to SETP21, e.g. nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "PART1" refers to the RNA, U6 small nuclear 806, pseudogene (Ensembl: ENSG00000202017), for example, to the sequence as defined in transcript no: ENST00000365147.1, specifically, to the nucleotide sequence as set forth in SEQ ID NO:8, which corresponds to the sequence of the above Ensembl Sequence of the PART1 transcript.

The term "PART1" also comprises nucleotide sequences showing a high degree of homology to PART1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:8.

In an embodiment the method comprises determining or receiving the transcription levels of at least one, preferably two, or all three transcripts selected from AC034234.1, RNU6-806P, and SETP21. The inventors found the most pronounce correlation between the expression levels of the transcripts AC034234.1, RNU6-806P, and SETP21, therefore preferably, one, two or all three of these transcripts are used in the method of the invention.

In an embodiment the outcome is chance of occurrence of castration resistant prostate cancer and/or chance of disease recurrence and/or chance of biochemical recurrence.

In an embodiment the prediction is further based on the expression levels of PDE4D, preferably PDE4D7. The inventors show that combining PDE4D, particularly PDE4D7 expression levels with non-coding RNA transcript levels of the PDE4D genomic region further improves the prediction. For example multiple transcription levels can be combined using a regression function.

In an embodiment the method comprises combining the gene expression profiles for two or more, for example, 2, 3, 4, 5, 6, 7 or all, of the transcripts with a regression function that had been derived from a population of prostate cancer subjects. Thus in an embodiment 2, 3, 4, 5, 6, 7 or all, of the transcripts selected from NDUFB4P2, MIR582, AC034234.1, RNU6-806P, AC109486.1, SETP21, and PART1 (and optionally further comprising the PDE4D or PDE4D7 transcription level) and combining the gene expression profiles with a regression function that had been derived from a population of prostate cancer subjects.

In an embodiment the prediction is further based on one or more clinical parameters obtained from the subject. In an embodiment the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. In an embodiment the determining of the outcome comprises combining the gene expression profile(s) for the one or more transcripts in the PDE4D genomic region and/or the DNA methylation levels in the PDE4D genomic region, and the one or more clinical parameters obtained from the subject with a regression function that had been derived from a population of prostate cancer subjects.

In an embodiment the method further comprises determining an optimal treatment strategy. In an embodiment the treatment strategy is a multi-modality therapy, preferably wherein the multi-modality therapy is selected from surgery followed by hormonal therapy with anti-androgens, radiation therapy hormonal therapy with anti-androgens, surgery followed by radiation therapy, and chemotherapy together with hormonal therapy with anti-androgens, if the predicted outcome is poor, and wherein the treatment strategy is selected from active surveillance, watchful waiting, focal therapy, neoadjuvant hormonal therapy with anti-androgens, cryoablation, surgery, and radiation therapy, if the predicted outcome is not poor (favorable).

In a third aspect the invention relates to a kit of parts comprising primers and optionally probes for the selective amplification and detection of at least one transcript selected from NDUFB4P2, MIR582, AC034234.1, RNU6-806P, AC109486.1, SETP21, and PART1, preferably selected from AC034234.1, RNU6-806P, and SETP21. It is understood that a PCR or qPCR kit can be used to determine the expression levels of the non-coding RNA transcripts, therefore the kit is preferably a PCR or a qPCR kit. The primers are preferably designed to specifically amplify a part of the at least one transcript selected from NDUFB4P2, MIR582, AC034234.1, RNU6-806P, AC109486.1, SETP21, and PART1. The probes are preferably designed to specifically detect at least one transcript selected from NDUFB4P2, MIR582, AC034234.1, RNU6-806P, AC109486.1, SETP21, and PART1. Methods to design and test primers and probes for these transcripts are known to the skilled person. Preferably the kit comprises primers and optionally probes for the selective amplification and detection of at least one, preferably two, three, four, five, six or all seven transcripts selected from NDUFB4P2, MIR582, AC034234.1, RNU6-806P, AC109486.1, SETP21

In a further aspect the invention relates to the use of the kit as broadly defined herein for predicting an outcome for a human prostate cancer patient, the use comprising: determining the expression level of the at least one transcript using the kit; and predicting the outcome based on the at least one expression level.

In an alternative embodiment the invention relates to a method of treating, preventing or ameliorating a prostate cancer patient in need thereof, the method comprising:
a) receiving information on the transcription level or determining the transcription level of at least one transcript from the genomic region of the human chromosome 5 bases 59,000,000 to 60,600,000 with the proviso that the transcript is not PDE4D or a specific PDE4D isoform, and/or receiving information on the DNA methylation level or determining the DNA methylation level in a genomic region of the human chromosome 5 bases 58,900,000 to 61,000,000, determined in a sample obtained from the patient,
b) comparing the transcription level and/or DNA methylation level to a reference, and
c) predicting a poor outcome if transcription levels are below the reference value and/or DNA methylation levels are above the reference value, or predicting a favourable outcome if transcription levels are above the reference value and/or DNA methylation levels are below the reference value; and

when the predicted outcome is poor providing a multi-modality therapy to the subject, and
when the predicted outcome is favorable providing a therapy selected from active surveillance, watchful waiting, focal therapy, neoadjuvant hormonal therapy with anti-androgens, cryoablation, surgery, and radiation therapy to the patient.

In an embodiment the multi-modality therapy is selected from surgery followed by hormonal therapy with anti-androgens, radiation therapy hormonal therapy with anti-androgens, surgery followed by radiation therapy, and chemotherapy together with hormonal therapy with anti-androgens.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

### EXAMPLES

### Example 1

### Reduced PDE4D7 expression in prostate cancer correlates with down-regulation of genomic elements within the up-stream PDE4D coding region on chromosome 5

### Methods

Two independent NGS transcriptomics expression data sets (n=533 and n=151) from PCa patient biopsy punch samples of surgically resected tumour tissue were used in this study. RNA sequencing and data processing was performed as previously described by van Strijp *et al.* (2019). Generation of normalized PDE4D transcript expression was performed by subtracting the RT-qPCR Cq of respective transcripts from the averaged reference gene RT-qPCR Cq. Normalised PDE4D5, PDE4D7, and PDE4D9 expression was transformed to the PDE4D5, PDE4D7, and PDE4D9 scores. To investigate the expression of genomic elements like miRNAs or pseudogenes on chromosome 5 in the region where PDE4D7 exons 1-3 are located (chr5:59,000,000 to chr5:60,500,000), we created a heatmap of the expression of respective transcripts of these genomic elements together with the level of PDE4D5, PDE4D7 and PDE4D9 expression. For this we transferred the PDE4D5, PDE4D7 and PDE4D9 scores as well as the transcript per million (TPM) expression values for the respective transcripts into z-scores on the gene level across all samples. As a result, all genes of the heatmap have a mean expression of 0 and a SD of 1.

### Results

Our analysis suggests a distinct correlation between PDE4D7 expression and that of a subset of lncRNAs, miRNAs and pseudogenes located between 60,000,000 and 60,500,000 base pairs on chromosome 5. Importantly, all trends observed are correlated between both independent datasets. Specifically, RNA transcripts located within the first 3 exons of PDE4D7 are coincidentally downregulated, with AC034234.1, RNU6-806P, AC109486.1, SETP21, PART1 and the entire PDE4D gene exhibiting a similar pattern of expression to PDE4D7 among samples. These genes all showed a p-value <0.05 between samples with high vs low PDE4D7 scores, alongside AC092343.1, AC008833.1, NDUFB4P2 and MIR582.

Whilst AC109486.1 exhibits this pattern, AC109486.2 and AC109486.3 show no expression in any sample, alongside AC016591.1. Interestingly, all samples with no expression are found in the region overlapping PART1 which exhibits similar expression to PDE4D7, revealing that this entire region is not deleted.

CAB39P1 showed almost a uniform lack of expression across all samples, however a minimal number of samples exhibited increased expression irrelevant to PDE4D7 level. AC016642.1, RAB3C, RPL5P15 and AC008852.1 revealed similar lack of expression but with more anomalous samples exhibiting altered expression. The most varied expression profiles were DEPDC1B, KRT8P31 and ELOVL7, as well as PDE4D5 and PDE4D9, reflecting no obvious correlation to PDE4D7 level. Of note here is that TMPRSS2-ERG negative patient samples show more pronounced downregulation of genomic elements than TMPRSS2-ERG positive samples, like that shown relative to PDE4D7 score.

Heat map of gene expression in PCa patient samples from datasets 1 n=533 and 2 n=151 ordered according to their PDE4D7 scores from high to low was generated (not shown). Gene expression values of the shown transcripts located in the genomic region of the PDE4D gene on chromosome 5 were determined by NGS RNA sequencing. Transcripts PDE4D, AC092343.1, AC008833.1, NDUFB4P2, MIR582, AC034234.1, RNU6-806P, AC109486.1, SETP21 and PART1 show p-value <0.05 in expression difference between samples with high vs low PDE4D7 score. Chromosome 5 genome alignment in PDE4D coding region between approx. 59,000,000 - 60,500,000bp from UCSC genome browser (GRCh38/hg38 Human Dec 2013 assembly), accessed on 14 April 2022 demonstrates that PDE4D7 isoform and these trsanscripts share the same genomic location. A aeat map of gene expression in PCa patient samples from datasets 1 (n=533) ordered according to their TMPRSS2-ERG fusion status was generated to show correlation (not shown).

### Discussion & Conclusion

Böttcher *et al.* (2016) noted that long isoforms of the PDE4D family are significantly downregulated during disease progression. However, as PDE4D5 and PDE4D9 are downregulated in both AS PCa and CRPC tissue whereas PDE4D7 is only diminished in CRPC, it represents a more specific measurement of disease progression. Other RNAs mapped to the PDE4D coding region also exhibiting PDE4D7-like downregulation are pseudogenes (SETP21, RNU6-806P), nucleotide sequences (AC034234.1 and AC109486.1) and long non-coding (lnc) RNAs [prostrate androgen-regulated transcript-1 (PART1)]. No function has been ascribed to the majority of these genes. We are the first to report the similarity in expression amongst various genes located within the PDE4D7 coding region of Chr5q12. Correlation between PDE4D7 level and TMPRSS2-ERG status has previously been identified, with upregulated PDE4D7 in TMPRSS2-ERG+ tumours and low-grade PCa. Given that the PDE4D7 gene contains an ERG binding site, it was speculated that PDE4D7 may be regulated via ERG transcription. Our data further support this, with TMPRSS2-ERG- samples indicating downregulation of the analysed genes akin to those identified upon stratification by PDE4D7 score. In summation, our data identifies the downregulation of an entire chromosomal region of Chr5q12 mapping to the PDE4D7 domain throughout PCa progression. This pinpoints not only driver genes but also passenger genes which may be a feature of carcinogenesis.

### Example 2

Cox proportional-hazards regression was used for each of the individual transcripts as well as a combination model to verify if transcripts are predictive. For the model, survival time is defined as time from surgery to biochemical recurrence. The results are summarized below:

### NDUFB4P2

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2573,695 |
| Chi-squared | 8,206 |
| DF | 1 |
| Significance level | P = 0.0042 |

### Coefficients and Standard Errors

| Covariate | b | SE | Wald | P | Exp(b) | 95% CI of Exp(b) |
|---|---|---|---|---|---|---|
| NDUFB4P2 | -0,4092 | 0,1754 | 5,4387 | 0,0197 | 0,6642 | 0.4709 to 0.9368 |

### Concordance

| | |
|---|---|
| Harrell's C-index | 0,532 |
| 95% Confidence interval | 0.511 to 0.553 |

### MIR582

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2574,137 |
| Chi-squared | 7,764 |
| DF | 1 |
| Significance level | P = 0.0053 |

### Coefficients and Standard Errors

| Covariate | b | SE | Wald | P | Exp(b) | 95% CI of Exp(b) |
|---|---|---|---|---|---|---|
| MIR582 | -0,2478 | 0,1078 | 5,2866 | 0,0215 | 0,7805 | 0.6319 to 0.9641 |

### Concordance

| | |
|---|---|
| Harrell's C-index | 0,537 |
| 95% Confidence interval | 0.517 to 0.558 |

### AC034234.1

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2560,273 |
| Chi-squared | 21,627 |
| DF | 1 |
| Significance level | P < 0.0001 |

### Coefficients and Standard Errors

| Covariate | b | SE | Wald | P | Exp(b) | 95% CI of Exp(b) |
|---|---|---|---|---|---|---|
| AC034234.1 | -0,6478 | 0,1935 | 11,2122 | 0,0008 | 0,5232 | 0.3581 to 0.7644 |

### Concordance

| | |
|---|---|
| Harrell's C-index | 0,583 |
| 95% Confidence interval | 0.556 to 0.610 |

### RNU6_806P

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2567,21 |
| Chi-squared | 14,69 |
| DF | 1 |
| Significance level | P = 0.0001 |

### Coefficients and Standard Errors

| Covariate | b | SE | Wald | P | Exp(b) | 95% CI of Exp(b) |
|---|---|---|---|---|---|---|
| RNU6_806P | -0,3928 | 0,1208 | 10,5785 | 0,0011 | 0,6752 | 0.5329 to 0.8555 |

### Concordance

| | |
|---|---|
| Harrell's C-index | 0,601 |
| 95% Confidence interval | 0.564 to 0.639 |

### AC109486.1

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2574,532 |
| Chi-squared | 7,369 |
| DF | 1 |
| Significance level | P = 0.0066 |

### Coefficients and Standard Errors

| Covariate | b | SE | Wald | P | Exp(b) | 95% CI of Exp(b) |
|---|---|---|---|---|---|---|
| AC109486.1 | -0,2201 | 0,08875 | 6,1524 | 0,0131 | 0,8024 | 0.6743 to 0.9549 |

### Concordance

| | |
|---|---|
| Harrell's C-index | 0,593 |
| 95% Confidence interval | 0.554 to 0.631 |

### SETP21

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2558,703 |
| Chi-squared | 23,198 |
| DF | 1 |
| Significance level | P < 0.0001 |

### Coefficients and Standard Errors

| Covariate | b | SE | Wald | P | Exp(b) | 95% CI of Exp(b) |
|---|---|---|---|---|---|---|
| SETP21 | -0,4863 | 0,1205 | 16,2893 | 0,0001 | 0,6149 | 0.4855 to 0.7787 |

### Concordance

| | |
|---|---|
| Harrell's C-index | 0,642 |
| 95% Confidence interval | 0.605 to 0.678 |

### PART1

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2572,562 |
| Chi-squared | 9,338 |
| DF | 1 |
| Significance level | P = 0.0022 |

### Coefficients and Standard Errors

| Covariate | b | SE | Wald | P | Exp(b) | 95% CI of Exp(b) |
|---|---|---|---|---|---|---|
| PART1 | -0,2531 | 0,08761 | 8,3434 | 0,0039 | 0,7764 | 0.6539 to 0.9219 |

### Concordance

| | |
|---|---|
| Harrell's C-index | 0,577 |
| 95% Confidence interval | 0.537 to 0.617 |

### Combination model

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2533,259 |
| Chi-squared | 48,641 |
| DF | 7 |
| Significance level | P < 0.0001 |

### Coefficients and Standard Errors

| Covariate | b | SE | Wald | P | Exp(b) | 95% CI of Exp(b) |
|---|---|---|---|---|---|---|
| NDUFB4P2 | -0,2969 | 0,1695 | 3,0679 | 0,0799 | 0,7431 | 0.5331 to 1.0360 |
| MIR582 | -0,1536 | 0,1057 | 2,1116 | 0,1462 | 0,8576 | 0.6972 to 1.0550 |
| AC034234.1 | -0,505 | 0,195 | 6,7066 | 0,0096 | 0,6035 | 0.4118 to 0.8844 |
| RNU6_806P | -0,05569 | 0,1546 | 0,1298 | 0,7187 | 0,9458 | 0.6986 to 1.2806 |
| AC109486.1 | 0,2503 | 0,1118 | 5,0144 | 0,0251 | 1,2845 | 1.0317 to 1.5991 |
| SETP21 | -0,4951 | 0,1518 | 10,6366 | 0,0011 | 0,6095 | 0.4526 to 0.8207 |
| PART1 | -0,0288 | 0,09767 | 0,08698 | 0,7681 | 0,9716 | 0.8023 to 1.1766 |

### Concordance

| | |
|---|---|
| Harrell's C-index | 0,661 |
| 95% Confidence interval | 0.626 to 0.697 |

### Combination model + PDE4D7

### Cases summary

| | | |
|---|---|---|
| Number of events a | 218 | 40,90% |
| Number censored b | 315 | 59,10% |
| Total number of cases | 533 | 100,00% |
| a BCR_class = 1 | | |
| b BCR_class = 0 | | |

### Overall Model Fit

| | |
|---|---|
| Null model -2 Log Likelihood | 2581,901 |
| Full model -2 Log Likelihood | 2512,977 |
| Chi-squared | 68,924 |
| DF | 8 |
| Significance level | P < 0.0001 |

### Coefficients and Standard Errors

| Covariate | b | | SE | Wald | | P | Exp(b) | |
|---|---|---|---|---|---|---|---|---|
| | 95% CI of Exp(b) | | | | | | | |
| NDUFB4P2 | -0,258 | | 0,1677 | 2,367 | | 0,1239 | 0,7726 | 0.5562 to 1.0732 |
| MIR582 | -0,1571 | 0,1084 | | 2,101 | | 0,1472 | 0,8546 | 0.6910 to 1.0569 |
| AC034234.1 | -0,4426 | 0,1872 | | 5,5903 | | 0,0181 | 0,6423 | 0.4451 to 0.9271 |
| RNU6_806P | -0,002786 | | 0,1484 | 0,0003524 | | 0,985 | 0,9972 | 0.7455 to 1.3339 |
| AC109486.1 | 0,2538 | | 0,1151 | 4,8589 | | 0,0275 | 1,2889 | 1.0285 to 1.6151 |
| SETP21 | -0,3369 | 0,1512 | | 4,9646 | | 0,0259 | 0,714 | 0.5309 to 0.9603 |
| PART1 | 0,1742 | | 0,09875 | 3,1128 | | 0,0777 | 1,1903 | 0.9809 to 1.4445 |
| PDE4D7 | -0,4203 | 0,09212 | 20,8212 | <0.0001 | 0,6568 | 0.5483 to 0.7868 | | |

### Concordance

| | |
|---|---|
| Harrell's C-index | 0,671 |
| 95% Confidence interval | 0.637 to 0.706 |

Kaplan-Meier curves were generated for the combination model and the combination model + PDE4D7 and depicted in Figs. 2 and 3.

**In conclusion** - each of the transcripts individually significantly predicted biochemical recurrence.

### Example 3 - Methylation in the PDE4D genomic region

Figs. 4 to 7 depict data of primary tumor samples (Fig. 4 and 5) and for the normal samples (Fig. 6 and 7). Shown on the y-axis are the genome positions (start to end) of the probe sets. On the x-axis is shown the so-called beta-values which is a measure between 0-1 where 0 means no methylation and 1 means complete methylation of the GC site that is measured by the respective probe. The box plots represent the beta values for the ca 500 primary tumor samples (Figs. 4 and 5) and the ca 50 normal prostate samples (Figs. 6 and 7). Also indicated are the next probe set(s) for the features of interest in the PDE4D region, these are the genomic features that have been measured for expression as well as the starting exon of the various PDE4D isoforms 1-9.

With the used technology it is evident that there are differences in terms of methylation across the PDE4D gene region between primary tumor samples and normal tissue. The region that is more towards the start of the PDE4D gene, where also the first exons of PDE4D7 are located (Fig. 5 and 7) is generally more methylation compared to the end of the gene (Fig. 4 and 6). All regions of the entire gene are generally more methylated in primary tumor samples compared to normal prostate tissue samples.

Statistical analysis was performed to check whether the beta level for a probe set is correlated with the PDE4D expression (this data is also existing for the TCGA samples). Also checked was the association of the beta levels for each probe set to the sample type (tumor vs normal), and to biochemical recurrence (recurrent vs non-recurrent). All this data is shown in the below Table. For the correlation to the PDE4D expression most probe sets are negatively correlated which means that the higher the PDE4D expression the lower is the level of methylation across most probe sets.

For the other two analysis on sample type and BCR there are only p-values. For the tissue type analysis there is strong association between many probe sets and the tissue type (normal vs tumor). For BCR there are some probe sets (e.g., those are located next to the starting exons of PDE4D7) that are significantly associated with disease relapse, however prediction accuracy is expected to improve by combining specific probes or basing the prediction on overall methylation of the PDE4D7 genomic region.

| **methylation probe set** | **PDE4D expression** | | **sample type (PT, NAT)** | | **BCR (biochemical recurrence)** |
|---|---|---|---|---|---|
| | *p_value pearson_r* | | *p_value* | | *p_value* |
| cg14482364 | 8,13E-14 0,32 | 7,69E-06 | | 2,44E-02 | |
| cg06046805 | 2,12E-06 -0,20 | 4,43E-07 | | 3,62E-01 | |
| cg14862385 | 1,48E-040,16 | 8,69E-06 | | 2,99E-02 | |
| cg09062288 | 2,43E-040,16 | 6,54E-05 | | 5,07E-02 | |
| cg07200957 | 3,84E-13 -0,31 | 4,04E-18 | | 6,21E-01 | |
| cg05602356 | 3,60E-10 -0,27 | 9,10E-24 | | 2,50E-01 | |
| cg03367519 | 4,35E-06-0,20 | 2,97E-22 | | 2,26E-01 | |
| cg17633020 | 2,42E-02-0,10 | 1,36E-25 | | 1,52E-01 | |
| cg18242030 | 3,79E-04-0,15 | 2,84E-19 | | 8,35E-02 | |
| cg27543403 | 2,13E-05-0,18 | 1,24E-09 | | 5,81E-01 | |
| cg09092093 | 1,26E-03-0,14 | 1,19E-11 | | 2,63E-01 | |
| cg25480082 | NA | NA | NA | | NA |
| cg16377679 | 8,62E-01-0,01 | 6,79E-04 | | 9,23E-01 | |
| cg16578883 | 6,80E-03-0,12 | 3,27E-04 | | 2,20E-01 | |
| cg26939607 | 7,50E-09 0,25 | 4,30E-04 | | 7,75E-02 | |
| cg07348982 | 2,65E-14 0,33 | 1,45E-06 | | 3,31E-02 | |
| cg05691871 | 4,48E-05 -0,18 | 1,38E-07 | | 6,32E-01 | |
| cg26817935 | 3,12E-10 -0,27 | 1,10E-02 | | 1,37E-01 | |
| cg17563336 | 5,31E-13-0,31 | 3,51E-03 | | 1,08E-01 | |
| cg23987137 | 6,91E-19 -0,39 | 3,30E-31 | | 7,34E-04 | |
| cg06974135 | 2,31E-11 0,29 | 6,57E-08 | | 4,40E-02 | |
| cg15834202 | 1,58E-13 0,32 | 1,24E-03 | | 2,20E-02 | |
| cg05992340 | 1,38E-01-0,06 | 2,21E-02 | | 7,72E-01 | |
| cg13112511 | 1,65E-04 0,16 | 1,00E+00 | | | 1,71E-01 |
| cg01174811 | 9,09E-010,00 | 2,12E-04 | | 4,06E-01 | |
| cg18804667 | 4,71E-01-0,03 | 1,00E+00 | | | 2,39E-01 |
| cg07267294 | 3,19E-03 -0,13 | 3,28E-26 | | 7,21E-01 | |
| cg07330169 | 7,93E-01-0,01 | 6,66E-06 | | 4,00E-01 | |
| cg12519013 | 3,53E-05 -0,18 | 4,85E-03 | | 5,79E-02 | |
| cg24741713 | 1,75E-01-0,06 | 5,08E-01 | | 4,11E-01 | |
| cg13397436 | 2,86E-04-0,16 | 1,73E-06 | | 1,55E-01 | |
| cg03653541 | 1,44E-12-0,31 | 8,90E-10 | | 2,33E-02 | |
| cg12400680 | 5,59E-16-0,35 | 1,58E-11 | | 2,52E-02 | |
| cg18689666 | 2,74E-08 -0,24 | 1,00E+00 | | | 4,25E-01 |
| cg08500112 | 4,52E-01-0,03 | 9,77E-01 | | 3,06E-01 | |
| cg17328171 | 4,76E-13 -0,31 | 1,94E-06 | | 4,22E-02 | |
| cg26800788 | 1,65E-02-0,10 | 1,26E-02 | | 5,68E-01 | |
| cg14784398 | 1,76E-01-0,06 | 8,82E-02 | | 2,51E-01 | |
| cg27583655 | 9,42E-04 0,14 | 1,08E-02 | | 2,01E-01 | |
| cg01817885 | NA | NA | NA | | NA |
| cg17010100 | NA | NA | NA | | NA |
| cg06469229 | 1,04E-21 -0,43 | 8,65E-24 | | 7,01E-02 | |
| cg03154690 | 4,73E-17 -0,37 | 2,22E-20 | | 3,11E-02 | |
| cg03323696 | 6,57E-07 -0,21 | 5,89E-22 | | 3,86E-01 | |
| cg03804272 | 1,07E-02 -0,11 | 2,36E-11 | | 1,95E-01 | |
| cg26078977 | 2,93E-01-0,05 | 5,25E-13 | | 3,48E-01 | |
| cg26870744 | 3,82E-01-0,04 | 1,07E-10 | | 4,27E-01 | |
| cg22706610 | 6,09E-01 -0,02 | 5,12E-11 | | 5,30E-01 | |
| cg16261871 | 2,09E-01-0,05 | 4,87E-09 | | 3,77E-01 | |
| cg11258089 | 6,46E-01-0,02 | 5,04E-10 | | 5,85E-01 | |
| cg07190535 | 4,59E-01-0,03 | 1,71E-10 | | 4,29E-01 | |
| cg24628676 | 7,98E-01-0,01 | 2,97E-10 | | 5,68E-01 | |
| cg08204702 | 8,22E-010,01 | 1,69E-10 | | 6,85E-01 | |
| cg08554295 | 3,94E-01 -0,04 | 1,43E-09 | | 4,61E-01 | |
| cg20473977 | 2,39E-02-0,10 | 3,92E-15 | | 7,63E-01 | |
| cg23094665 | 3,59E-01-0,04 | 6,02E-03 | | 2,73E-01 | |
| cg15410418 | 1,61E-06-0,21 | 4,54E-06 | | 9,57E-02 | |
| cg10487428 | 1,74E-18-0,39 | 2,34E-09 | | 4,35E-02 | |
| cg22495322 | NA | NA | NA | | NA |
| cg17287998 | NA | NA | NA | | NA |
| cg04580929 | NA | NA | NA | | NA |
| cg13404427 | 9,63E-14 0,32 | 5,41E-05 | | 4,85E-02 | |
| cg21023952 | 3,27E-010,04 | 1,05E-05 | | 4,50E-01 | |
| cg09712066 | 2,29E-010,05 | 2,95E-05 | | 4,13E-01 | |
| cg18704047 | 6,28E-010,02 | 5,42E-06 | | 4,29E-01 | |
| cg26353176 | 3,35E-05 0,18 | 4,64E-06 | | 6,99E-01 | |
| cg05141014 | 2,37E-04 0,16 | 4,04E-05 | | 4,02E-01 | |
| cg24844211 | 1,52E-040,16 | 2,88E-09 | | 5,84E-01 | |

### References

1. Henderson DJP, Houslay MD, Bangma CH, Hoffmann R. Creating a potential diagnostic for prostate cancer risk stratification (InformMDxTM) by translating novel scientific discoveries concerning cAMP degrading phosphodiesterase-4D7 (PDE4D7). Clin Sci. 2019 [cited 2019 Oct 2];133(2):269-86. Available from: https://doi.org/10.1042/CS20180519
2. Merkle D, Hoffmann R. Roles of cAMP and cAMP-dependent protein kinase in the progression of prostate cancer: Cross-talk with the androgen receptor. Cell Signal [Internet]. 2011;23(3):507-15. Available from: http://dx.doi.org/10.1016/j.cellsig.2010.08.017
3. Baillie GS. Compartmentalized signalling: Spatial regulation of cAMP by the action of compartmentalized phosphodiesterases. FEBS J. 2009;276(7):1790-9.
4. Henderson DJP, Byrne A, Dulla K, Jenster G, Hoffmann R, Baillie GS, et al. The cAMP phosphodiesterase-4D7 (PDE4D7) is downregulated in androgen-independent prostate cancer cells and mediates proliferation by compartmentalising cAMP at the plasma membrane of VCaP prostate cancer cells. Br J Cancer [Internet]. 2014 Mar 11 [cited 2019 Oct 2];110(5):1278-87. Available from: http://www.nature.com/articles/bjc201422
5. Van Strijp D, De Witz C, Heitkötter B, Huss S, Bögemann M, Baillie GS, et al. The association of the long prostate cancer expressed PDE4D transcripts to poor patient outcome depends on the tumour's TMPRSS2-ERG fusion status. Prostate Cancer. 2019;2019.
6. Alves de Inda M, van Strijp D, den Biezen-Timmermans E, van Brussel A, Wrobel J, van Zon H, et al. Validation of Cyclic Adenosine Monophosphate Phosphodiesterase-4D7 for its Independent Contribution to Risk Stratification in a Prostate Cancer Patient Cohort with Longitudinal Biological Outcomes. Eur Urol Focus. 2018 Apr 1;4(3):376-84.
7. Böttcher R, Dulla K, Van Strijp D, Dits N, Verhoef EI, Baillie GS, et al. Human PDE4D isoform composition is deregulated in primary prostate cancer and indicative for disease progression and development of distant metastases. Oncotarget [Internet]. 2016 Oct 25 [cited 2019 Oct 2];7(43):70669-84. Available from: http://www.ncbi.nlm.nih.gov/pubmed/27683107
8. Böttcher R, Henderson DJP, Dulla K, Van Strijp D, Waanders LF, Tevz G, et al. Human phosphodiesterase 4D7 (PDE4D7) expression is increased in TMPRSS2-ERG-positive primary prostate cancer and independently adds to a reduced risk of post-surgical disease progression. Br J Cancer. 2015;113(10):1502-11.
**The attached Sequence Listing, entitled 2023PF00510 SEQ LIST, is incorporated herein by reference, in its entirety.**

## Claims

1. A computer implemented method for predicting an outcome for a human prostate cancer patient, the method comprising
a) receiving information on the transcription level of at least one transcript from the genomic region of the human chromosome 5 bases 59,000,000 to 60,600,000 with the proviso that the transcript is not PDE4D or a specific PDE4D isoform, and/or receiving information on the DNA methylation level in a genomic region of the human chromosome 5 bases 58,900,000 to 61,000,000, determined in a sample obtained from the patient,
b) comparing the transcription level and/or DNA methylation level to a reference, and
c) predicting a poor outcome if transcription levels are below the reference value and/or DNA methylation levels are above the reference value, or predicting a favorable outcome if transcription levels are above the reference value and/or DNA methylation levels are below the reference value.

2. A method for determining an outcome for a human prostate cancer patient, the method comprising
a) determining the transcription level of at least one transcript from the genomic region of the human chromosome 5 bases 59,000,000 to 60,600,000 with the proviso that the transcript is not PDE4D or a specific PDE4D isoform, and/or determining the DNA methylation level in a genomic region of the human chromosome 5 bases 58,900,000 to 61,000,000, determined in a sample obtained from the patient,
b) comparing the transcription level and/or DNA methylation level to a reference, and
c) predicting a poor outcome if transcription levels are below the reference value and/or DNA methylation levels are above the reference value, or predicting a favorable outcome if transcription levels are above the reference value and/or DNA methylation levels are below the reference value.

3. The method according to claim 1 or 2 wherein the human prostate cancer patient is a patient with primary localized disease, locally or regionally advanced disease, or metastatic disease.

4. The method according to any one of the preceding claims wherein the at least one transcript is selected from NDUFB4P2, MIR582, AC034234.1, RNU6-806P, AC109486.1, SETP21, and PART1, preferably wherein the method comprises determining or receiving the transcription levels of at least two, preferably three, four, five, six, or all seven transcripts selected from NDUFB4P2, MIR582, AC034234.1, RNU6-806P, AC109486.1, SETP21, and PART1.

5. The method according to any one of the preceding claims wherein the method comprises determining or receiving the transcription levels of at least one, preferably two, or all three transcripts selected from AC034234.1, RNU6-806P, and SETP21.

6. The method according to any one of the preceding claims wherein the outcome is chance of occurrence of castration resistant prostate cancer and/or chance of disease recurrence and/or chance of biochemical recurrence.

7. The method according to any one of the preceding claims wherein the prediction is further based on the expression levels of PDE4D, preferably PDE4D7.

8. The method according to any one of the preceding claims wherein the determining of the prediction of the outcome comprises combining the gene expression profiles for two or more, for example, 2, 3, 4, 5, 6, 7 or all, of the transcripts with a regression function that had been derived from a population of prostate cancer subjects.

9. The method according to any one of the preceding claims wherein the prediction is further based on one or more clinical parameters obtained from the subject.

10. The method according to claim 9, wherein the clinical parameters comprise one or more of: (i) a prostate-specific antigen (PSA) level; (ii) a pathologic Gleason score (pGS); iii) a clinical tumor stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

11. The method as defined in claim 9 or 10, wherein the determining of the prediction of the radiotherapy response comprises combining the gene expression profile(s) for the one or more PDE4D7 correlated genes and the one or more clinical parameters obtained from the subject with a regression function that had been derived from a population of prostate cancer subjects.

12. The method according any one of the preceding claims wherein the method further comprises determining an optimal treatment strategy.

13. The method according to claim 12 wherein the treatment strategy is a multi-modality therapy, preferably wherein the multi-modality therapy is selected from surgery followed by hormonal therapy with anti-androgens, radiation therapy hormonal therapy with anti-androgens, surgery followed by radiation therapy, and chemotherapy together with hormonal therapy with anti-androgens, if the predicted outcome is poor, and wherein the treatment strategy is selected from active surveillance, watchful waiting, focal therapy, neoadjuvant hormonal therapy with anti-androgens, cryoablation, surgery, and radiation therapy, if the predicted outcome is favorable.

14. A kit of parts comprising primers and optionally probes for the selective amplification and detection of at least one transcript selected from NDUFB4P2, MIR582, AC034234.1, RNU6-806P, AC109486.1, SETP21, and PART1, preferably selected from AC034234.1, RNU6-806P, and SETP21.

15. Use of the kit according to claim 14 for predicting an outcome for a human prostate cancer patient, the use comprising:
determining the expression level of the at least one transcript using the kit; and
predicting the outcome based on the at least one expression level.
